# EUROPEAN PATENT APPLICATION

(11) **EP 2 672 411 A1**
(43) Date of publication of application: **11.12.2013**
(21) Application number: 12170795.4
(22) Date of filing: 05.06.2012
(51) Int. Cl.: G06F 19/00

(54) **Digital image storage system and human body data matching algorithm for medical aesthetic application**

(71) Applicant: Chen, Chun-Leon, New Taipei City 24764 (TW)
(72) Inventor: Chen, Chun-Leon, New Taipei City 24764 (TW); Cha, Jennifer, Las Vegas NV 89117 (US)
(74) Representative: K & H Bonapat

(57) **Abstract**

A human body data matching algorithm for medical aesthetic application includes the step of providing a human body image acquisition unit (1), the step of using the human body image acquisition unit (1) to obtain a digital 3D image data of the body of a person during an optimal lifetime of the person, the step of storing the digital 3D image data in a data storage bank (wl), the step of fetching the digital 3D image data from the data storage bank (wl) when going to perform a medical aesthetic surgery, and the step of e) matching the digital 3D image data for reference in the medical aesthetic surgery to avoid communication errors between the patient and the surgeon.

## Description

### BACKGROUND OF THE INVENTION

### 1. Field of the Invention:

The present invention relates to medical aesthetic technologies and more particularly, to a digital image storage system for medical aesthetic application. The invention relates also to a human body data matching algorithm for medical aesthetic application.

### 2. Description of the Related Art:

Some medical institutions provide medicare-related hanks for storing, for example, stem cell, sperm, ovum, and/or cord blood for future health care use.

However, these medicare-related storage items are obtained from the human body for a respective specific purpose.

To some surgical surgery or cosmetic surgery, these medicare-related hanks have no real contribution.

A medical surgery or cosmetic surgery may be performed for the purpose of making the human body back to the optimal physical conditions, for example, skin color, body proportion, face length, and etc. However, it may sometimes be difficult to accurately describe the optimal physical conditions, leading to a communication error between the patient and the surgeon and patient's unsatisfaction of the surgical result.

### SUMMARY OF THE INVENTION

The present invention has been accomplished under the circumstances in view. It is one object of the present invention to provide a human body data matching algorithm for medical aesthetic application, which enables a surgeon to review the patient's early body data for matching before performing a medical or cosmetic surgery to avoid any communication errors with the patient.

To achieve this and other objects of the present invention, a human body data matching algorithm for medical aesthetic application, comprises the steps of: a) providing a human body image acquisition unit comprising a 3D image sensor for scanning a human body based on a predetermined reference point to obtain a complete data of soft tissue, hard tissue and skin color of every part of the human body and then rendering the data into a digital 3D image data; b) using the human body image acquisition unit to obtain a digital 3D image data of the body of a person during a human body optimal lifetime of the person; c) storing the digital 3D image data in a data storage bank; d) fetching the digital 3D image data from the data storage bank when going to perform a medical aesthetic surgery; and e) matching the digital 3D image data and then performing the medical aesthetic surgery.

To achieve this and other objects of the present invention, a digital image storage system for medical aesthetic application comprises a human body image acquisition unit comprising a digital 3D image sensor for scanning the body of a person based on a predetermined reference point during an optimal lifetime of the person to obtain a complete data of soft tissue, hard tissue and skin color of every part of the body of the person and then rendering the data into a digital 3D image data, a data storage unit for storing the digital 3D image data, and a display unit for displaying the digital 3D image data for reference in a medical aesthetic surgery for future reconstruction, study of how individual growth pattern, forensic or recreational purpose.

### BRIEF DESCRIPTION OF THE DRAWINGS

FIG. 1 is block diagram of a digital image storage system for medical aesthetic application in accordance with the present invention.
FIG. 2 is a human body data matching algorithm flow chart in accordance with the present invention.

### DETAILED DESCRIPTION OF THE PREFERRED EMBODIMENT

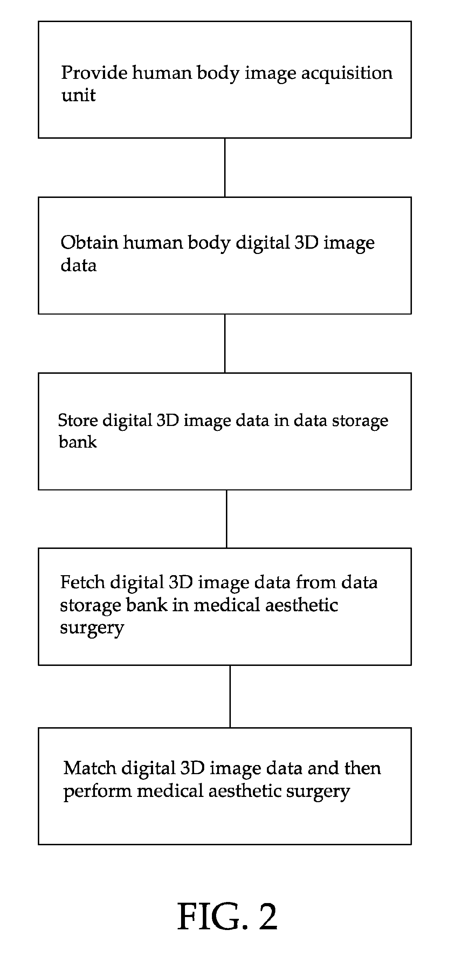

Referring to FIG. 2, a human body data matching algorithm in accordance with the present invention includes the steps of:
a) providing a human body image acquisition unit comprising a 3D image sensor for scanning a human body based on a predetermined reference point to obtain a complete data of soft tissue, hard tissue and skin color of every part of the human body and then rendering the data into a digital 3D image data;
b) using the human body image acquisition unit to obtain a digital 3D image data of the body of a person during an optimal lifetime of the person;
c) storing the digital 3D image data thus obtained in a data storage bank;
d) fetching the digital 3D image data from the data storage bank before performing a medical aesthetic surgery for future reconstruction, study of how individual growth pattern, forensic or recreational purpose; and
e) matching the digital 3D image data and then performing the medical aesthetic surgery.

In actual practice, the human body optimal lifetime is, for example, in the age range 18-28 years. Within this age range, the human body image acquisition unit is used to a digital 3D image data of the body of a person. This digital 3D image data includes the data of the skeleton, tissues and outer skin of the human body. This digital 3D image data is then stored in a data storage bank for permanent preservation subject to the personal data, such as birthday, name, personal ID, etc. When a patient having his(her) early body data stored in the data storage bank is going to receive a medical or aesthetic surgery, the doctor can fetch the storage digital 3D image data of the body of the patient from the data storage bank and display the data on a display screen for reference in the surgical operation.

Following rise in age, the teeth may wear down, the skin color may change, and the skeleton may waste away. If one wishes to receive a medical or aesthetic surgery in order to take back the optimal conditions of the body as when he(she) was young, the digital 3D image data of his(her) body stored in the data storage bank in an early day can be fetched and provided to the doctor for direct reference, avoiding communication errors and medication mistakes.

For example, when one is going to take a skin whitening treatment, the digital 3D image data of the skin of the body obtained and stored during the optimal lifetime can be fetched from the data storage bank for reference to achieve an optimal treatment result.

Further, when the invention is used for dental application, a dentist can identify the original height of the lower jaw for the determination of the sinus lift elevation in the dental implant subject to the respective storage digital 3D image data, achieving optimal implant results.

Further, when taking a knee replacement surgery, the surgeon can obtain the early storage digital 3D image data of the patient from the data storage bank for reference in determining the height of the artificial knee joint, achieving optimal knee replacement surgical results.

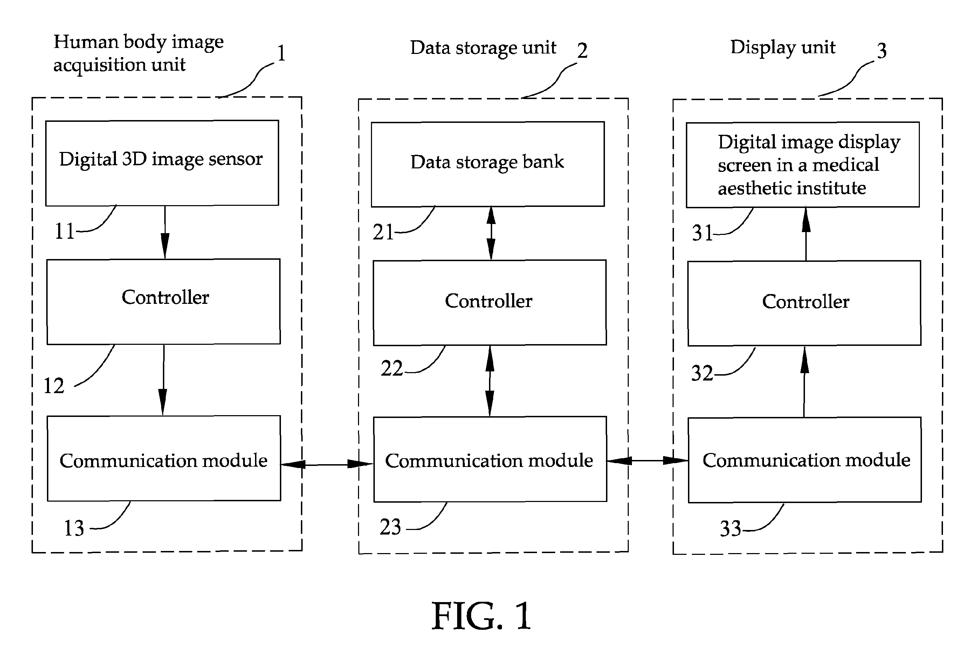

The invention also provides a digital image storage system for medical or aesthetic applications. As illustrated in FIG. 1, the digital image storage system comprises a human body image acquisition unit **1,** a data storage unit **2,** and a display unit **3.** The human body image acquisition unit **1** comprises a digital 3D image sensor **11** for full body scan, magnetic resonance imaging (MRI), ultrasound imaging, computed tomography (CT) scan, and intra and extra oral scan. The 3D digital image sensor **11** is controllable to scan the body of a person based on a predetermined reference point during the person's optimal lifetime, thereby obtaining a complete data of soft tissue, hard tissue and skin color of every part of the person's body and then to render the data into a digital 3D image data. This digital 3D image data is then stored in the data storage unit **2.** Thus, when a patient having his(her) early body data stored in the data storage unit **2** is going to receive a medical or aesthetic surgery, his(her) early body data (digital 3D image data) can be fetched from the data storage unit **2** and then displayed on the display unit **3** for reference in the surgical operation.

The aforesaid human body image acquisition unit **1** further comprises a controller **12** electrically coupled with the digital 3D image sensor **11** for converting the digital 3D image data produced by the digital 3D image sensor **11** into a modulated digital 3D image data subject to a predetermined modulation format, and a communication module **13** electrically coupled to the controller **12** for transmitting the modulated digital 3D image data to the data storage unit **2** for storage.

The data storage unit **2** comprises a communication module **23** for receiving the modulated digital 3D image data transmitted by the communication module **13** of the human body image acquisition unit **1,** a controller **22** electrically coupled with the communication module **23** for demodulating the modulated digital 3D image data received by the communication module **23,** and a data storage bank **21** electrically coupled with the controller **22** and controlled by the controller **22** to store the demodulated digital 3D image data.

The display unit **3** comprises a digital image display screen **31** installed in a medical aesthetic institute, a communication module **33,** and a controller **32** electrically coupled with the digital image display screen **31** and the communication module **33** for controlling the communication module **33** to communicate with the communication module **23** of the data storage unit **2** and to further fetch a storage digital 3D image data from the data storage bank **21** for display on the digital image display screen **31.**

Although a particular embodiment of the invention has been described in detail for purposes of illustration, various modifications and enhancements may be made without departing from the spirit and scope of the invention. Accordingly, the invention is not to be limited except as by the appended claims.

## Claims

1. A digital image storage system for medical aesthetic application, comprising:
a human body image acquisition unit (1) for scanning the body of a person based on a predetermined reference point during an optimal lifetime of said person to obtain a complete data of soft tissue, hard tissue and skin color of every part of the body of said person and then rendering the data into a digital 3D image data;
a data storage unit (2) for storing said digital 3D image data (1); and
a display unit (3) for displaying said digital 3D image data for reference in a medical aesthetic surgery for future reconstruction, study of how individual growth pattern, forensic or recreational purpose.

2. The digital image storage system as claimed in claim 1, wherein said human body image acquisition unit (1) comprises a digital 3D image sensor (11) for full body scan, magnetic resonance imaging (MRI), ultrasound imaging, computed tomography (CT) scan, and intra and extra oral scan, a controller (12) for converting said digital 3D image data produced by said digital 3D image sensor (11) into a modulated digital 3D image data subject to a predetermined modulation format, and a communication module (13) controllable by the controller of said human body image acquisition unit (1) to transmit the modulated digital 3D image data to said data storage unit for storage.

3. The digital image storage system as claimed in claim 2, wherein said data storage unit (2) comprises a communication module (23) for receiving the modulated digital 3D image data transmitted by the communication module (13) of said human body image acquisition unit (1), a controller (22) for demodulating the modulated digital 3D image data received by the communication module (23) of said data storage unit (2), and a data storage bank (21) controlled by the controller (22) of said data storage unit (2) to store the demodulated digital 3D image data.

4. The digital image storage system as claimed in claim 3, wherein said display unit (3) comprises a digital image display screen (31) installed in a medical aesthetic institute, a communication module (33), and a controller (32) for controlling the communication module (33) of said display unit (3) to communicate with the communication module (23) of said data storage unit (2) and to further fetch a storage digital 3D image data from said data storage bank (21) for display on said digital image display screen (31).

5. A human body data matching algorithm for medical aesthetic application, comprising the steps of:
a) providing a human body image acquisition unit (1) for scanning a human body based on a predetermined reference point to obtain a complete data of soft tissue, hard tissue and skin color of every part of the human body and then rendering the data into a digital 3D image data;
b) using said human body image acquisition unit (1) to obtain a digital 3D image data of the body of a person during a human body optimal lifetime of said person;
c) storing said digital 3D image data in a data storage bank (21);
d) fetching said digital 3D image data from said data storage bank (21) when going to perform a medical aesthetic surgery; and
e) matching said digital 3D image data and then performing the medical aesthetic surgery.

6. The human body data matching algorithm for medical aesthetic application as claimed in claim 5, wherein said human body image acquisition unit (1) comprises a digital 3D image sensor (11) for full body scan, magnetic resonance imaging (MRI), ultrasound imaging, computed tomography (CT) scan, and intra and extra oral scan, a controller (12) for converting said digital 3D image data produced by said digital 3D image sensor (11) into a modulated digital 3D image data subject to a predetermined modulation format, and a communication module (13) controllable by the controller (12) of said human body image acquisition unit (1) to transmit the modulated digital 3D image data to said data storage unit (2) for storage.
